Europäisches Patentamt

European Patent Office

Office européen des brevets

(19)

(11) Veröffentlichungsnummer: **0 008 450**
**B1**

## EUROPÄISCHE PATENTSCHRIFT

(12)

(45) Veröffentlichungstag der Patentschrift:
31.03.82

(21) Anmeldenummer: 79103031.5

(22) Anmeldetag: 20.08.79

(51) Int. Cl.³: **A 61 B 5/00**

(54) Urinmessgerät.

(30) Priorität: 24.08.78  DE 7825225 U

(43) Veröffentlichungstag der Anmeldung:
05.03.80 Patentblatt 80/5

(45) Bekanntmachung des Hinweises auf die Patenterteilung:
31.03.82 Patentblatt 82/13

(84) Benannte Vertragsstaaten:
AT BE CH DE FR GB IT LU NL SE

(56) Entgegenhaltungen:
CH-A-559 352
DE-A-1 928 737
FR-A-2 239 981
GB-A-1 128 186
US-A-3 362 400
US-A-3 831 446
US-A-3 906 935

(73) Patentinhaber: Intermedicat GmbH, Gerliswilstrasse 45,
CH-6020 Emmenbrücke (CH)

(72) Erfinder: Griesel, Karl-Heinz, Oberer Weinberg,
D-3508 Meisungen (DE)

(74) Vertreter: von Kreisler, Alek, Dipl.-Chem. et al,
Deichmannhaus am Hauptbahnhof, D-5000 Köln 1 (DE)

EP 0 008 450 B1

## Urinmeßgerät

Die Erfindung bezieht sich auf ein Urinmeßgerät zum Auffangen, Sammeln und Messen des Urins katheterisierter Patienten, das aus einem durchsichtigen, starren Meßbehälter besteht, der durch Trennwände in mehrere, nebeneinanderliegende, graduierte Kammern unterteilt ist, wobei in die erste Kammer ein Zulauf mündet und alle Kammern oben gegeneinander offen und mit ihren unteren Kammerauslaßöffnungen über Ventilmittel an einen Ablauf angeschlossen sind, mit dem ein Urinbeutel in Verbindung steht, und wobei hinter der letzten Kammer ein mit dem Ablauf verbundener Überlauf vorgesehen ist.

Ein bekanntes Urinmeßgerät (FR-A-2 239 981) besteht aus einem graduierten Meßbehälter aus festem, transparentem Werkstoff, der nur eine Kammer enthält und an den ein flexibler Urinbeutel angeschlossen ist. Der aus dem Katheter des Patienten ablaufende Urin gelangt über einen Zuleitungsschlauch in den Meßbehälter und wird hier gesammelt. Die durch den Urin verdrängte Luft verläßt den Meßbehälter über ein bakteriendichtes, flüssigkeitsdruckbeständiges Filter. Nach einer gewissen Zeit wird die Menge des gesammelten Urins an der Skala des Meßbehälters abgelesen und der Urin in den unter dem Meßbehälter befindlichen Urinbeutel abgelassen, wozu ein Ventil am Boden des Meßbehälters geöffnet wird. Der Urinbeutel faßt mehrere Füllungen des Meßbehälters. Falls bis zum Entleeren des Meßbehälters mehr Urin produziert wird als der Meßbehälter faßt, wird die überschüssige Menge über einen Überlauf unter Umgehung des Ventils direkt in den Urinbeutel geleitet. Urinbeutel und Meßbehälter sind flüssigkeitsdicht miteinander verbunden. Ist der Urinbeutel gefüllt, wird er gegen einen neuen Urinbeutel ausgetauscht.

Im oberen Bereich der Skala ist bei diesem Urinmeßgerät eine Ablesung mit der gewünschten Genauigkeit oftmals nicht möglich. Eine Verbesserung der Ablesegenauigkeit im unteren Bereich wird durch Spreizung der Skala erreicht, die sich durch eine besondere Formgebung des Meßbehälters ergibt. Dies führt zu einer beträchtlichen Höhe des Urinmeßgerätes, so daß häufig trotz ordnungsgemäßer Befestigung des Urinmeßgerätes am Krankenbett der Urinbeutel auf dem Boden hängt, was unerwünscht ist.

Um bei einem Urinmeßgerät eine genauere Ablesbarkeit der Skala zu erreichen, ist das eingangs erläuterte Urinmeßgerät mit zwei nebeneinanderliegenden senkrechten Kammern entwickelt worden (CH-A-559 352). Dabei ist in die Kammerauslaßöffnung jeder Kammer jeweils ein gesondertes Ventil eingesetzt worden, und die Ventile müssen einzeln betätigt werden. Dies ist umständlich und zeitraubend und begrenzt die praktisch verwendbare Anzahl von Kammern des Meßbehälters. Zwischen dem Überlauf und dem Ablauf ist ein Auffangtrichter angeordnet und mit dem über eine Steckverbindung an den Meßbehälter angeschlossenen Urinbeutel hat das Urinmeßgerät eine beträchtliche, unzweckmäßige Höhe.

Der Erfindung liegt die Aufgabe zugrunde, ein Urinmeßgerät so auszubilden, daß es sich einfach handhaben läßt, eine genaue Ablesung an jeder Stelle der Skala erlaubt und bei ausreichendem Volumen niedrige Bauhöhe aufweist.

Diese Aufgabe wird dadurch gelöst, daß zwischen die Kammerauslaßöffnungen aller Kammern und den Ablauf des Meßbehälters ein einziges Ventil in den Meßbehälter eingebaut ist, das alle Kammern gleichzeitig einzeln absperrt und gleichzeitig einzeln öffnet.

Aufgrund des Vorhandenseins nur eines einzigen Ventils für alle Kammern läßt sich der Meßbehälter unabhängig von der Anzahl der Kammern schnell und einfach öffnen und schließen, so daß er beliebig in so viele Kammern unterteilt werden kann, wie zur Erzielung einer für eine ausreichend genaue Ablesung erforderlichen Spreizung der Skala notwendig sind. Durch die mehrfache Unterteilung des Meßbehälters und die den Kammern einzeln zugeordneten Skalen sinkt der Ablesefehler proportional zu der Verkleinerung der Kammergrundflächen. Da das Ventil jede Kammern einzeln absperrt, wird verhindert, daß der Urin nach dem Prinzip kommunizierender Röhren in allen Kammern gleich hoch steht, und die Vorteile der Spreizung der Skala wieder aufgehoben werden. Die Verwendung nur eines einzigen Ventils hat außer der Vereinfachung der Handhabung des Urinmeßgerätes den Vorteil, daß auch eine Verf        ung von Meßergebnissen durch unsachg       ße Bedienung praktisch ausgeschlossen ist. Die Unterteilbarkeit des Meßbehälters in eine Vielzahl von Kammern ist ferner günstig, weil sie ermöglicht, seine Bauhöhe ohne Verminderung seines Fassungsvermögens niedrig zu halten, indem er verbreitert wird. Die Gesamthöhe des Urinmeßgerätes mit angehängtem Urinbeutel wird auf diese Weise herabgesetzt, so daß der Urinbeutel bei ordnungsgemäßer Befestigung des Urinmeßgerätes an einem Krankenbett mit sicherem Abstand oberhalb des Fußbodens hängt.

Um zu vermeiden, daß durch den Ablauf des Meßbehälters mitgerissene Luft in den Urinbeutel gelangt, die sich in dem Urinbeutel staut und seine vollständige Füllung verhindert, ist vorgesehen, daß zwischen dem Ausgang den Ventils und dem Ausgang des Überlaufs in dem Ablauf eine Drosselstelle ausgebildet ist. Die Lufteinschleppung wird um so sicherer verhindert, je kleiner die Drosselstelle im Verhältnis zu der maßgebenden Öffnung des Ventils ist. Diese Ausbildung verursacht durch die Massenträgheit des austretenden Urins in dem Ablauf zwischen dem Meßbehälter und dem Urinbeutel einen leichten Stau, der bewirkt, daß der Urin in den Überlauf hineinläuft und ihn gegen von hier eventuell einströmende Luft abschließt. Auf Grund der Verhinderung von Luftstauungen in dem Urinbeutel können einfache und preiswerte Urinbeutel ohne Entlüftungsöffnung benutzt werden.

In vorteilhafter Ausgestaltung der Erfindung sind die Ausgänge des Ventils und des Überlaufs in T-förmiger Leitungsführung an den zwischen ihnen befindlichen Ablauf angeschlossen. Bei dieser Anordnung unterstützt die Massenträgheit des austretenden Urins die Drosselwirkung der Drosselstelle im Ablauf, so daß sich der Überlauf noch leichter mit Urin füllt.

Eine weitere Erleichterung der Handhabung des Urinmeßgeräts ergibt sich durch die beanspruchte Konstruktion des Ventils, das sich durch begrenzte Axialverschiebung eines Kolbens einfach bedienen läßt.

Die Funktion des Urinmeßgerätes wird im übrigen dadurch verbessert, daß auf beiden Seiten des Ablaufs an dem Meßbehälter in der Symmetrieebene Haken zur Anhängung von Aufhängeösen des Urinbeutels angeordnet sind, der mittels Steckverbindung an den Ablauf lösbar angeschlossen ist. Meßbehälter und Urinbeutel werden mit Hilfe der Haken fest, jedoch lösbar miteinander verbunden. Bei lotrecht hängendem Meßbehälter ist gewährleistet, daß auch der Urinbeutel lotrecht hängt und ein einwandfreier Austritt des Urins aus dem Ablauf des Meßbehälters in den Urinbeutel stattfindet. Ferner bewirkt dieses Merkmal, daß bei Anhängung des Urinbeutels an die beiden Haken des Meßbehälters zwangsläufig auch die Steckverbindung zusammengefügt wird, so daß das Urinmeßgerät bei angehängtem Urinbeutel zuverlässig funktionsbereit ist.

Eine weitere Vereinfachung und Verbesserung der Benutzung des Urinmeßgerätes ergibt sich dadurch, daß eine an sich bekannte Punktionsstelle zur Probenentnahme mittels einer Spritze in der Vorderwand des Meßbehälters im bodennahen Bereich der ersten Kammer und/oder in dem quergerichteten Schenkel eines T-Rohrstückes vorgesehen ist, das in einen den Zulauf des Meßbehälters mit einem Katheter verbindenden Schlauch eingesetzt ist.

Ein Ausführungsbeispiel der Erfindung ist in der Zeichnung schematisch dargestellt. Das erfindungsgemäße Gerät vereinigt in sich folgende Funktionen:

— Auffangen des Urins katheterisierter Patienten
— Messen des in einem bestimmten Zeitraum produzierten Urins
— Sammeln des Urins
— Entnahmemöglichkeit von Urinproben

Fig. 1 zeigt eine Seitenansicht eines Urinmeßgerätes, an das ein Urinbeutel angehängt ist,

Fig. 2 ist ein Querschnitt durch eine Punktionsstelle zur Probeentnahme am Meßbehälter mittels einer handelsüblichen Einmalspritze,

Fig. 3 ist ein Längsschnitt eines geschlossenen Ventils und

Fig. 4 zeigt einen Längsschnitt des geöffneten Ventils.

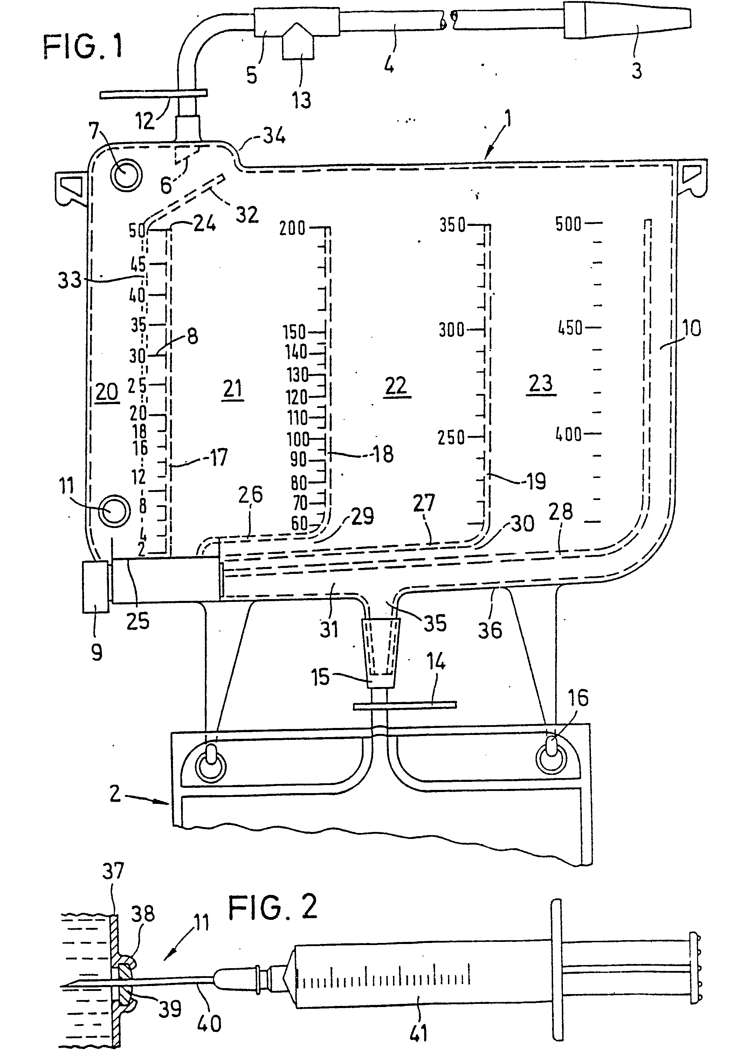

Gemäß Fig. 1 besteht ein erfindungsgemäßes Urinmeßgerät aus einem Meßbehälter 1 und einem auswechselbaren Urinbeutel 2. Ein Ansatz 3 ist mit dem entsprechenden Gegenstück des nicht dargestellten Katheters verbunden. Aus dem Katheter gelangt der Urin über einen Zuleitungsschlauch 4, ein T-Stück 5 zur Probenentnahme und einen Tropfer 6 in den Meßbehälter 1, wobei die verdrängte Luft aus einer Entlüftung 7 entweicht. Die in einer bestimmten Zeiteinheit gesammelte Urinmenge kann an Skalen 8 abgelesen werden. Ein Ventil 9 dient zum Ablassen des Urins in den Urinbeutel 2. Falls der Meßbehälter 1 gefüllt ist, bevor das Ventil 9 geöffnet wird, läuft der überschüssige Urin durch einen Überlauf 10 direkt in den Urinbeutel 2. Probeurin kann an den Punktionsstellen 11 oder 13 entnommen werden. Ist der Urinbeutel 2 gefüllt, kann er nach Schließen einer Klemme 14 und Trennen einer Steckverbindung 15 von den Haken 16 abgenommen und durch einen neuen Beutel ersetzt werden. Der gefüllte Urinbeutel kann anschließend durch Öffnen der Klemme 14 problemlos entleert und dann vernichtet werden.

Um Ablesefehler klein zu halten bzw. sie besonders im oberen Bereich des Meßbehälters 1 noch zu vermindern, ist der Meßbehälter durch mehrere, beispielsweise drei senkrechte Zwischenwände 17, 18, 19 unterteilt. Die Zwischenwände begrenzen Kammern 20, 21, 22, 23. Der Urin erreicht zuerst die Kammer 20. Ist diese gefüllt, läuft der Urin über den oberen Rand 24 in die zweite Kammer 21 usf. Durch die Unterteilung des Meßbehälters in mehrere Kammern sinkt der Ablesefehler proportional zu der Verkleinerung der Grundflächen 25, 26, 27 und 28.

Zum Ablassen läuft der Urin aus den ersten beiden Kammern 20, 21 direkt zu dem Ventil 9 und von dort durch einen gemeinsamen Ausgang 31 in den Urinbeutel 2. Das Ventil 9 muß jede Kammer einzeln absperren. Es darf nicht in dem gemeinsamen Ausgang 31 angeordnet sein, da der Urin sonst nach dem Prinzip der kommunizierenden Gefäße in allen Kammern gleich hoch stehen würde, womit die Wirkung der Zwischenwände wieder aufgehoben würde.

In der oberen Zulauföffnung im Deckel des Meßbehälters 1 ist ein Tropfer 6 angeordnet, der die retrograde Keimwanderung in den Zuleitungsschlauch 4 verringern soll. Die Keimwanderung wird zusätzlich unterbunden, wenn es gelingt, die innere Oberfläche des Meßbehälters 1 in der Umgebung des Tropfers 6 trocken zu halten, da trockene Kunststoffoberflächen kein Nährboden für die in Frage kommenden Keime sind. Die Umgebung des Tropfers 6 kann durch zurückspritzenden Urin befeuchtet werden, wenn die Tropfen auf die Flüssigkeitsoberfläche fallen. Um das Zurückspritzen zu verhindern, ist eine Prallplatte 32 vorgesehen, auf die der Tropfen nicht rechtwinklig wie auf die Flüssigkeitsoberfläche, sondern in einem spitzen Winkel trifft. Wenn der Winkel richtig gewählt ist, überwiegen die Adhäsionskräfte zwischen Tropfen und Prallplatte 32 und der Tropfen wird auf der Prallplatte 32 festgehalten, von wo er über einen anschließenden senkrechten Teil 33 ohne Spritzer abläuft, so daß die Umgebung des Tropfers trocken bleibt.

Eine zweite Maßnahme zur Verhinderung der Keimwanderung in den Zuleitungsschlauch 4 besteht in der Präparierung der Umgebung des Tropfers 6 mit einem keimtötenden Mittel. Hierzu können beispielsweise Phenole, Aldehydkondensate verwendet werden. Um die präparierte Fläche und gleichzeitig die Tropfenfallstrecke zu vergrößern, ist das Meßbehälter-Oberteil zu einem Dom 34 ausgebildet.

Die Einschleppung von Luft in den Urinbeutel 2 wird durch die in Fig. 1 dargestellte Anordnung des Ausgangs 31 des Ventils 9, des Überlaufs 10 und der Steckverbindung 15 verhindert. Der innere Querschnitt der Steckverbindung 15 ist als Drosselstelle 35 etwas kleiner gehalten als der Querschnitt des geöffneten Ventils 9. Durch diese Dimensionierung entsteht in der Steckverbindung 15 ein leichter Stau, der bewirkt, daß der Urin in den Überlauf 10 hineinläuft und ihn gegen von hier eventuell einströmende Luft abschließt. Wie in Fig. 1 dargestellt ist, bilden der waagerechte Ausgang 36 des Überlaufs 10 und der Ausgang 31 des Ventils 9 die waagerechten Schenkel und die Steckverbindung 15 den senkrechten Schenkel eines großen T. Bei dieser Anordnung unterstützt die Massenträgheit des austretenden Urins die Drosselwirkung der Drosselstelle 35, so daß sich der Überlauf 10 noch leichter mit Urin füllt.

Für die sterile Entnahme von Untersuchungsurin sind alternativ Punktionsstellen 11 und 13 vorgesehen. Die Punktionsstelle 11 ist in der Vorderwand 37 im Bereich der ersten Kammer 20 angebracht. Der in dieser Kammer zulaufende Urin verdrängt den bereits vorhandenen Urin in nachfolgende Kammern, so daß hier immer eine frische Probe entnommen werden kann. Die Punktionsstelle 11 besteht aus einer gummielastischen Membran 39, die mittels einer Fassung 38 fest und dicht mit der Vorderwand 37 verbunden ist. Für die Probenentnahme wird die Membran mit einer Kanüle 40 punktiert und die Probe in eine Spritze 41 gesaugt. Wird die Kanüle 40 aus dem Membran 39 herausgezogen, verschließt sich die Einstichstelle infolge der inneren Spannung der Membran automatisch wieder.

In ähnlicher Weise arbeitet die Punktionsstelle 13 im T-Stück 5. Soll hier eine Probe entnommen werden, wird die Klemme 12 am Zuleitungsschlauch 4 geschlossen, bis sich im Zuleitungsschlauch 4 oder in der Blase des Patienten eine ausreichende Probemenge gesammelt hat. Beide Entnahmemöglichkeiten haben den Vorteil, daß das Urinmeßgerät zur Probenentnahme nicht geöffnet werden muß und die Sterilität dadurch gewahrt bleibt.

Das Ventil 9 besteht aus einem Gehäuse 45 mit einer zylindrischen Bohrung 46, die an beiden Enden offen ist und einen Kolben 47 enthält. Der Kolben 47 ist in der Bohrung 46 axial verschiebbar; er ist zylindrisch gestaltet und weist fünf Umfangsrillen 48 auf, die von unterbrechungslosen ringförmigen Dichtlippen 49 begrenzt sind. Der Kolben 47 und die Dichtlippen 49 können aus gummielastischem Material hergestellt sein. Zum Öffnen und Schließen des Ventils 9 kann der Kolben 47 mit Hilfe von zwei Druckknöpfen 50, 51 axial verschoben werden, die in die offenen Enden des Gehäuses 45 eingesetzt sind.

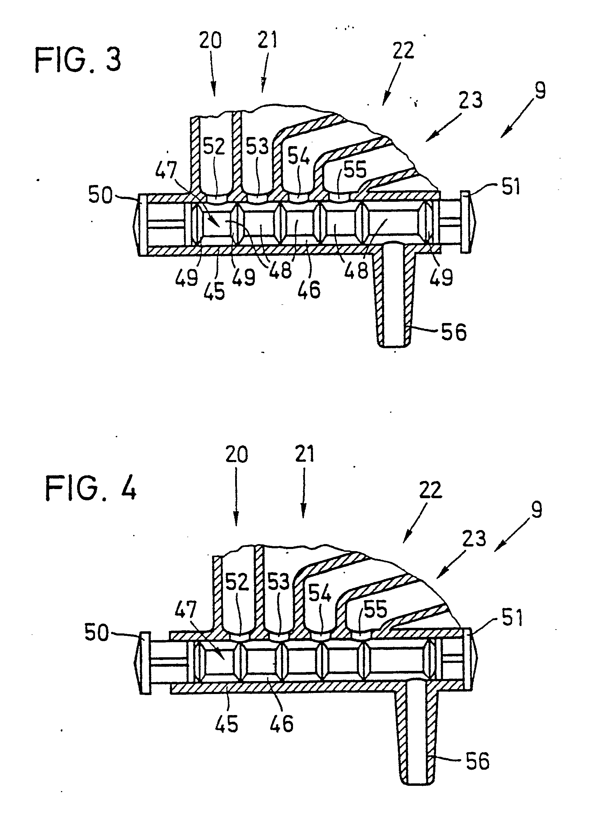

Vier Kammerauslaßöffnungen 52, 53, 54 und 55 in der Wand des Gehäuses 45 verbinden die Bohrung 46 bzw. die Räume zwischen den Dichtlippen 49 mit den Kammern 20, 21, 22, 23 des Meßbehälters 1. Bei geschlossenem Ventil 9 (Fig. 3) ist jede Kammerauslaßöffnung 52, 53, 54, 55 von je zwei Dichtlippen 49 begrenzt, während bei geöffnetem Ventil 9 (Fig. 4) je eine Dichtlippe 49 in eine Kammerauslaßöffnung ragt und so Überläufe bis zu einem Auslaß 56 entstehen. Der Auslaß 56 ist an den Ausgang 31 angeschlossen, durch den Urin in den Urinbeutel 2 läuft, wenn das Ventil 9 geöffnet ist.

## Patentansprüche

1. Urinmeßgerät zum Auffangen, Sammeln und Messen des Urins katheterisierter Patienten, das aus einem durchsichtigen starren Meßbehälter (1) besteht, der durch Trennwände (17, 18, 19) in mehrere, nebeneinanderliegende, graduierte Kammern (20, 21, 22, 23) unterteilt ist, wobei in die erste Kammer (20) ein Zulauf (6) mündet und alle Kammern (20, 21, 22, 23) oben gegeneinander offen und mit ihren unteren Kammerauslaßöffnungen (52, 53, 54, 55) über Ventilmittel (9) an einen Ablauf angeschlossen sind, mit dem ein Urinbeutel (2) in Verbindung steht und wobei hinter der letzten Kammer (23) ein mit dem Ablauf verbundener Überlauf (10) vorgesehen ist, dadurch gekennzeichnet, daß zwischen die Kammerauslaßöffnungen (52, 53, 54, 55) aller Kammern (20, 21, 22, 23) und den Ablauf des Meßbehälters (1) ein einziges Ventil (9) in den Meßbehälter (1) eingebaut ist, das alle Kammern (20, 21, 22, 23) gleichzeitig einzeln absperrt und gleichzeitig einzeln öffnet.

2. Urinmeßgerät nach Patentanspruch 1, dadurch gekennzeichnet, daß zwischen dem Ausgang (31) des Ventils (9) und dem Ausgang (36) des Überlaufes (10) in dem Ablauf eine Drosselstelle (35) ausgebildet ist.

3. Urinmeßgerät nach Patentanspruch 1 oder 2, dadurch gekennzeichnet, daß die Ausgänge (31; 36) des Ventils (9) und des Überlaufes (10) in T-förmiger Leitungsführung an den zwischen ihnen befindlichen Ablauf angeschlossen sind.

4. Urinmeßgerät nach einem der Patentansprüche 1 bis 3, dadurch gekennzeichnet, daß das Ventil (9) sich an einer Seite des Bodens des Meßbehälters (1) befindet und ein Gehäuse (45) aufweist, das mit einem Auslaß (56) und mit den Kammerauslaßöffnung (52, 53, 54, 55) versehen ist, die die Kammern (20, 21, 22, 23) mit einer zylindrischen Bohrung (46) in dem Gehäuse (45) und mit dem Auslaß (56) verbinden, und daß in der Bohrung (46) ein Kolben (47) untergebracht ist, der mit Abstand angeordnete, ringförmige Dichtlippen (49) trägt und begrenzt axial so verschiebbar ist, daß in Schließstellung des Kolbens (47) jede Kammerauslaßöffnung (52, 53, 54, 55) von zwei Dichtlippen (49) begrenzt ist und in Öffnungsstellung des Kolbens (47) je eine Dichtlippe (49) sich unter einer Kammerauslaßöffnung (52, 53, 54, 55) befindet, so daß Überläufe zum Auslaß (56) entstehen.

5. Urinmeßgerät nach einem der Patentansprüche 1 bis 4, dadurch gekennzeichnet, daß auf beiden Seiten des Ablaufs an dem Meßbehälter (1) in der Symmetrieebene Haken (16) zur Anhängung von Aufhängeösen des Urinbeutels (2) angeordnet sind, der mittels Steckverbindung (15) an den Ablauf lösbar angeschlossen ist.

6. Urinmeßgerät nach einem der Patentansprüche 1 bis 5, dadurch gekennzeichnet, daß eine an sich bekannte Punktionsstelle (11) zur Probenentnahme mittels einer Spritze in der Vorderwand (37) des Meßbehälters (1) im bodennahen Bereich der ersten Kammer (20) und/oder in dem quergerichteten Schenkel eines T-Rohrstückes (5) vorgesehen ist, das in einen den Zulauf des Meßbehälters (1) mit einem Katheter verbindenden Zuleitungsschlauch (4) eingesetzt ist.

## Claims

1. Urine measuring instrument for receiving, collecting and measuring urine from catheterized patients comprising a transparent rigid measuring container (1) divided by separating walls (17, 18, 19) into several side-by-side graduated chambers (20, 21, 22, 23), an inlet (6) ending in the first chamber (20) and all chambers (20, 21, 22, 23) being open to one another at the top and being connected with their lower chamber outlet openings (52, 53, 54, 55) via valve means (9) to a discharge communicating with an urine bag (2), an overflow (10) connected to the discharge being provided behind the last chamber (23), characterized in that between the chamber outlet openings (52, 53, 54, 55) of all chambers (20, 21, 22, 23) and the discharge of the measuring container (1) there is mounted one sole valve (9) in the measuring container (1) which valve simultaneously locks individually all chambers (20, 21, 22, 23) and simultaneously opens them individually.

2. Urine measuring instrument according to claim 1, characterized in that between the exit (31) of the valve (9) and the exit (36) of the overflow (10) a throttle point (35) is formed in the discharge.

3. Urine measuring instrument according to claim 1 or 2, characterized in that the exits (31; 36) of the valve (9) and of the overflow (10) are connected in a T-shaped line to the discharge between them.

4. Urine measuring instrument according to one of the preceding claims 1 through 3, characterized in that the valve (9) disposed at one side of the bottom of the measuring container (1) has a housing (45) provided with an outlet (56) and with chamber outlet openings (52, 53, 54, 55) connecting the chambers (20, 21, 22, 23) with a cylindrical bore (46) in the housing (45) and with the outlet (56) and that the bore (46) houses a piston (47) which carries spaced annular sealing lips (49) and which is axially displaceable to a limited extent so that in closure position of the piston (47) each chamber outlet opening (52, 53, 54, 55) is limited by two sealing lips (49) while in opening position of the piston (47) one sealing lip each (49) is below a chamber outlet opening (52, 53, 54, 55) thus forming overflows to the outlet (56).

5. Urine measuring instrument according to one of the preceding claims 1 to 4, characterized in that at both sides of the discharge, hooks (16) are arranged at the measuring container (1) in the plane of symmetry to suspend loops of the urine bag (2) which is connected detachably to the discharge by means of a plug-type connector (15).

6. Urine measuring instrument according to one of the preceding claims 1 to 5, characterized in that a puncture point (11) known per se, for sample taking by means of a syringe is provided in the front wall (37) of the measuring container (1) in the zone close to the bottom of the first chamber (20) and/or in the transverse leg of a T-shaped pipe piece (5) which is mounted in a feed tube (4) connecting the inlet of the measuring container (1) to a catheter.

## Revendications

1. Appareil de mesure d'urine pour recevoir, collecter et mesurer l'urine de patients munis de cathéters, l'appareil se composant d'un récipient de mesure (1) rigide et transparent, qui est divisé par des cloisons (17, 18, 19) en plusieurs chambres graduées (20, 21, 22, 23) placées l'une à côté de l'autre, un orifice (6) d'entrée débouchant dans la première chambre (20) et toutes les chambres (20, 21, 22, 23) étant supérieurement en communication l'une avec l'autre et étant reliées par leurs orifices inférieurs de décharge (52, 53, 54, 55) et par l'intermédiaire d'une valve (9) à un conduit d'évacuation qui est relié à la poche d'urine (2), un trop-plein (10) relié au conduit de décharge étant placé en arrière de la dernière chambre (23), caractérisé en ce qu'il est prévu entre les orifices de décharge (52, 53, 54, 55) de toutes les chambres (20, 21, 22, 23) et le conduit de décharge du récipient de mesure (1) une seule valve (9) dans le récipient de mesure (1), cette valve assurant l'obturation individuelle simultanée et l'ouverture individuelle simultanée de toutes les chambres (20, 21, 22, 23).

2. Appareil de mesure d'urine selon la revendication 1, caractérisé en ce qu'il est prévu entre la sortie (31) de la valve (9) et la sortie (36) du trop-plein (10), dans le conduit de décharge, une zone d'étranglement (35).

3. Appareil de mesure d'urine selon l'une des revendications 1 ou 2, caractérisé en ce que les sorties (31, 36) de la valve (9) et du trop-plein (10) sont reliées au conduit de décharge par un guide en forme de T situé entre elles.

4. Appareil de mesure d'urine selon l'une quelconque des revendications 1 à 3, caractérisé en ce que la valve (9) est placée d'un côté du fond du récipient de mesure (1) et comporte un corps (45) qui est pourvu d'un orifice de sortie (56) et d'orifices de décharge de chambres (52, 53, 54, 55), qui relient les chambres (20, 21, 22, 23) à un trou cylindrique (46) ménagé dans le corps (45) et à l'orifice de sortie (56) et en ce qu'un piston (47) est logé dans le trou (46), porte des lèvres annulaires d'étanchéité (49) espacées les unes des autres et est déplaçable par translation axiale limitée de manière que, dans la position de fermeture du piston (47), chaque orifice de décharge de chambre (52, 53, 54, 55) soit délimité par deux lèvres d'étanchéité (49) alors que, dans la position d'ouverture du piston (47), une lèvre d'étanchéité respective (49) est placées en dessous d'un orifice de décharge de chambre (52, 53, 54, 55), de sorte qu'il s'établit des trop-pleins vers l'orifice de sortie (56).

5. Appareil de mesure d'urine selon l'une quelconque des revendications 1 à 4, caractérisé en ce qu'il est prévu des deux côtés du conduit de décharge, sur le récipient de mesure (1) et dans le plan de symétrie, des crochets (16) servant à la suspension d'oreilles de la poche dúrine (2), qui est reliée de façon séparable au conduit de décharge par une liaison emboîtable (15).

6. Appareil de mesure d'urine selon l'une quelconque des revendications 1 à 5, caractérisé en ce qu'une zone de ponction de type connu (11), utilisable pour un prélèvement d'échantillon à l'aide d'une seringue, est prévue dans la paroi avant (37) du récipient de mesure (1) dans la zone proche du fond de la première chambre (20) et/ou dans la branche d'orientation perpendiculaire d'un raccord tubulaire en T (5), qui est engagé dans un flexible d'admission (4) assurant la liaison de l'entrée du récipient de mesure (1) avec un cathéter.

FIG.1

FIG. 2

FIG. 3

FIG. 4